# EUROPEAN PATENT SPECIFICATION

(11) **EP 3 502 247 B1**
(45) Date of publication and mention of the grant of the patent: **19.11.2025**
(21) Application number: 19156967.2
(22) Date of filing: 14.05.2015
(51) Int. Cl.: C12N 11/02, C12N 11/08, C12P 7/6458, C12N 9/20

(54) **PROCESS FOR IMMOBILIZATION OF A LIPASE**
VERFAHREN ZUR IMMOBILISIERUNG EINER LIPASE
PROCÉDÉ D'IMMOBILISATION D'UNE LIPASE

(30) Priority: 20.05.2014 EP 14169180
(43) Date of publication of application: 26.06.2019
(62) Divisional of application: 15724572.1
(73) Proprietor: Bunge Loders Croklaan B.V., 1521 AZ Wormerveer (NL)
(72) Inventor: Bhaggan, Krishnadath, 1521 AZ Wormerveer (NL); Ma, Jun, 1521 AZ Wormerveer (NL); Miorini, Chiara, 34077 Ronchi dei Legionari (Gorizia) (IT); Taran, Viktoria, 2512 AM Den Haag (NL)
(74) Representative: Potter Clarkson

(56) References cited:
- EP-A1- 2 251 428
- EP-A2- 0 882 797
- WO-A1-94/28118
- WO-A1-99/15689
- WO-A2-02/090560
- US-B1- 6 605 452
- NEVENA, Z ET AL: "Immobilization of lipase from Candida rugosa on Sepabeads: the effect of lipase oxidation by periodates", BIOPROCESS BIOSYST ENG., vol. 34, 2011, pages 803 - 810, XP019938990
- HANDAYANI NURRAHMI ET AL: "Immobilization of Mucor miehei Lipase onto Macroporous Aminated Polyethersulfone Membrane for Enzymatic Reactions.", MEMBRANES 12 APR 2012, vol. 2, no. 2, 12 April 2012 (2012-04-12), pages 198 - 213, XP002791058, ISSN: 2077-0375

## Description

### Technical Field

The present invention relates to process for immobilizing a lipase on a support having a functional amino group, a process for producing a triglyceride composition using said immobilized lipase and to the use of the lipase in transesterification reactions.

### Background Art

Lipases (E.C. 3.1.1.3), belonging to the group of enzymes, catalyse specifically ester bonds in tri-, di-, and mono-acylglycerols to glycerol and fatty acids. They further catalyse other reactions such as interesterifications, esterifications, acidolysis, alcoholysis and aminolysis. The high costs of lipases make enzymatic processes economically unattractive. Immobilization of the lipases is a way to increase the industrial susceptibility of lipases and allows recovery of the lipase protein. Lipases can be immobilized on different supports applying various ways of pretreatment of the support or the lipase.

Nevena et al. (NEVENA, Z. Immobilization of lipase from Candida rugosa on Sepabeads: the effect of lipase oxidation by periodates. Bioprocess Biosyst Eng. 2011, no.34, p.803-810.) describes the use of certain Sepabeads^{®} having either amino functional groups or epoxy groups as suitable support for the immobilization of a non-specific lipase from Candida rugosa. Sepabeads^{®} having amino functional groups needed activation with glutaraldehyde or periodate to show improved activity

Palomo et al. (PALOMO, Jose M, et al. Interfacial adsorption of lipases on very hydrophobic support (octadecyl-Sepabeads): immobilization, hyperactivation and stabilization of the open form of lipases. Journal of Molecular Catalysis B: Enzymatic. 2002, vol.19, no.20, p.279-286.) tested the immobilization of various lipases on very hydrophobic support such as octadecyl-Sepabead^{®}.

WO 02/090560 is directed to the production of 1,2-diacylated glycerophospholipids and describes the immobilisation of a phospholipase on Duolite A 568 in the presence of sorbitan monooleate.

WO 94//28118 relates to lipases immobilized on hydrophobic, dry porous support materials such as Accurel EP100.

WO 99/015689 is directed to a surfactant-coated lipase complex immobilized onto an insoluble matrix prepared by a two step immobilization method. US6605452-B1 describes a lipase preparation containing a surfactant-coated lipase complex immobilized onto an insoluble matrix, wherein the surfactant may be a fatty acid polyol ester such as sorbitan monostearate.

Enzyme activity is vulnerable to immobilizations regents such as glutaraldehyde or immobilization support. To secure enzyme stability and activity after immobilization of the enzyme, often non-lipase proteins are added such as hen egg album or bovin serum albumin. However, these animal proteins are known to cause allergic reactions.

There remains a need for a simplified immobilization method without additional activation of the support and the right choice of support which will remain lipase activity and stability such as thermostability to enable the production of commercially relevant triglyceride compositions.

### Summary of invention

The objective of the present invention is to provide an immobilization process wherein pre-activation of the support could be avoided and if indeed a hydrophobic support such as Sepabeads^{®} having octadecyl groups (EC-OD) are able to perform transesterification reaction to obtain products of commercial importance. Another aim of the present invention was to provide an immobilization process avoiding treatment with non- lipase protein such as animal derived albumin to secure activity and stability of the lipase to produce triglyceride compositions.

Described herein is a process for immobilizing a lipase on a support containing a functional amino group in the presence of a surface-active material. The term functional amino group refers to an amino group which is engaged in interacting with or binding to the lipase and optionally, the support.

The present invention provides a process for producing a triglyceride by enzymatic transesterification by using a lipase, which is immobilized on a support having a functional amino group according to claim 1.

Also provided by the invention in another aspect is the use of the immobilized lipase producing a triglyceride fat composition comprising at least 15% by weight OPO according to claim 7.

The support having a functional amino group can be any support having an amino group such as amino-epoxy, or alkyl amino having a carbon chain of C1-C24, preferably C2-C10. The support comprises a methacrylic polymer. Preferably the polymer forms a matrix.

A preferred support of the present invention contains a functional alkylamino group such as ethyl amino or hexyl amino.

The mechanism of action between the support and the lipase is either by ionic interaction or chemical binding, wherein the ionic interaction is preferred.

The surfactant can be formed from sugars, (both mono-di-and polysaccharides), polyols (e.g. sorbitan and sorbitol) or polyethylene glycols having molecular weight from 350 to 35000, such as PEG s 600, 1500, 4000. Very suitable non-ionic surfactants are polyoxyethylene sorbitan C8-C24 fatty acid esters, in particular those derived from lauric acid, such as Tween 20^{®} or derived from oleic acid such as Tween 80^{®}.

The surfactant concentration in the aqueous solution should be sufficient to ensure effective loading of the support by the enzyme. Very good results were obtained by applying an aqueous solution with a surfactant concentration of at least 0.01 wt%, preferably 0.01-10, most preferably 0.1-5 wt.%.

An ideal amount of lipase in g to support in g is between 1-20 wt.% by weight, preferably 5-15% by weight.

The contact times applied can vary between wide ranges. Suitably, however, contact times between 1 and 72 hours are applied.

The aqueous lipase solution has preferable a concentration between 1 to 20 g/I.

Although the lipase enzyme can be any prior art 1,3-specific lipase, a preference is expressed for a lipase which is selected from 1) 1,3-specific lipases from *Rhizomucor miehei and Rhizopus oryzae and Thermomyces lanuginosus 2)* lipases from *Penicillium camembertii* specific for the hydrolysis of partial glycerides, preferably Amano G, and 3) lipases specific for the hydrolysis of esters or triglycerides, preferably a lipase from *Candida rugosa .* In particular preferred is a 1,3- specific lipase from *Rhizopus oryzae* such as Lipase D from Amano.

Immobilization of the lipase can be performed in many different ways. Suitably, the contact between support, lipase and/or surfactant is performed as a batch process, as a continuous process in a fixed bed, as a continuous process in a fluidized bed or in a continuously stirred tank, while the contacting is performed with a continuous motion of the lipase solution.

The immobilized lipase described herein can be applied in any enzymatic conversion process, such as hydrolysis of triglycerides, diglycerides or esters, but also the esterification or transesterification of fatty acids or diglycerides or triglycerides.

Preferably, the symmetrical triglycerides of the general formula ABA, are OPO or SOS, wherein O is oleic acid, P is palmitic acid and S is a saturated fatty acid selected from palmitic acid and stearic acid. A particular preferred triglyceride composition of the invention comprises at least 15% by weight OPO.

Triglyceride fats and oils are important commercial products and are used extensively in, for example, the food industry. Some triglycerides are nutritionally important and the triglyceride 1,3-dioleoyl-2-palmitoyl glyceride (OPO) is known to be an important component of human milk fat.

### Examples

The following non-limiting examples illustrate the invention and do not limit its scope in any way. In the examples and throughout this specification, all percentages, parts and ratios are by weight unless indicated otherwise.

### Example 1: Various Sepabeads^{®} with aqueous Lipase D preparation

Preparation of the lipase solutions: Seven lipase solutions were prepared according to Table 1. Sample N°7 was the control sample. All reagents were mixed at 150 rpm at room temperature between 3 to 24 hours and then centrifuged to receive the immobilized lipase as a pellet.

**Table 1**

| Sample, N° | Sepabeads^{®} (functional group) | Amount of Sepabeads in g | Lipase in g |
|---|---|---|---|
| 1 | EC-HA (Hexamethylami no) | 1.5 | 0.12 in 70 ml |
| 2 | EC-OD (Octadecyl) | 1.5 | 0.12 in 70 ml |
| 3 | EC-BU (Butyl) | 1.5 | 0.12 in 70 ml |
| 4 | EC-HFA (Amino-Epoxy) | 1.5 | 0.12 in 70 ml |
| 5 | EC-EA (Ethylamino) | 1.5 | 0.12 in 70 ml |
| 6 | EC-EP (Epoxy) | 1.5 | 0.12 in 70 ml |
| 7 | No support | 0 | 0.18 in 75 ml (equal to 0.12 in 70 ml) |

### Example 2 (comparative)

The acidolysis reaction was performed at 60 °C with all seven lipase preparations using the following acidolysis assay:
1 g Immobilized enzyme (use the pellet after centrifugation)
35 g PO stearin fraction (Feedstock)
49 g Oleic acid
0.126 g H₂O

Composition Feedstock to be found in Table 2.

**Table 2**

| | Feedstock |
|---|---|
| Carbon number | |
| C48 | 62.1 |
| C50 | 24.3 |
| C52 | 8.6 |
| C54 | 1.9 |
| C56 | 0.0 |

The carbon number was determined by GC according to AOCS Ce 5.86.

Table 3 provides the results of the various acidolysis reaction of feedstock after 24 h

**Table 3**

| | HA | OD | BU | HFA | EA | EP | Control |
|---|---|---|---|---|---|---|---|
| Carbon number | | | | | | | |
| C48 | 59.9 | 60.1 | 60.0 | 57.4 | 60.3 | 60.1 | 60.3 |
| C50 | 25.4 | 25.3 | 25.3 | 26.8 | 25.1 | 25.3 | 25.2 |
| C52 | 9.2 | 9.2 | 9.2 | 10.2 | 9.1 | 9.2 | 9.1 |
| C54 | 2.1 | 2.1 | 2.2 | 2.5 | 2.1 | 2.1 | 2.1 |
| C56 | 0.2 | 0.1 | 0.2 | 0.2 | 0.2 | 0.2 | 0.2 |

After 24 h nearly no product OPO or OOP (C52) was produced for all lipase preparations

### Example 3

70 ml of the lipase preparation of Example 1 was mixed with 2.4 g hen egg albumin, 0.65 g Tween 20^{®} and 1.5 g of the respective supports. The acidolysis reaction was preformed according to example 2.

Table 4 shows the results after acidolysis (24 hours) by using various sepabeads with aqueous lipase D solution in the presence of hen egg albumin and TWEEN 20.

**Table 4**

| | HA | OD | BU | HFA | EA | EP |
|---|---|---|---|---|---|---|
| Carbon number | | | | | | |
| C48 | 7.2 | 9.5 | 61.2 | 6.9 | 7.2 | 61.4 |
| C50 | 29.3 | 31.3 | 24.8 | 29.0 | 25.0 | 24.7 |
| C52 | 41.5 | 41.5 | 8.8 | 42.8 | 42.3 | 8.8 |
| C54 | 20.4 | 16.9 | 2.0 | 20.4 | 20.7 | 1.9 |
| C56 | 0.5 | 0.4 | 0.0 | 0.5 | 0.4 | 0.0 |

### Example 4

70 ml of the lipase preparation of Example 1 was mixed with 250 mg PEG 1500, 0.65 g Tween 20^{®} and 1.5 g of the respective supports. The acidolysis reaction was preformed according to example 2. As comparison immobilization on polypropylene (Accurel) under same reaction conditions was performed.

Table 5 shows the results after acidolysis (24 hours) by using various sepabeads with aqueous lipase D solution in the presence of PEG 1500 and Tween 20^{®}.

**Table 5**

| | HA | OD | HFA | EA | Accurel |
|---|---|---|---|---|---|
| Carbon number | | | | | |
| C48 | 6.7 | 61.9 | 6.8 | 6.8 | 58.2 |
| C50 | 27.3 | 24.4 | 27.6 | 27.3 | 25.2 |
| C52 | 42.4 | 8.5 | 42.5 | 42.4 | 10.0 |
| C54 | 22.5 | 1.9 | 22.0 | 22.4 | 2.3 |
| C56 | 0.2 | 0.0 | 0.5 | 0.4 | 4.1 |

### Example 5

Multiple usage of Lipase D immobilized on support EC-HA.

70 ml of the lipase preparation of Example 1 was mixed with 30 mg PEG 600, 0.65 g Tween 20^{®} and 1.5 g of support EC-HA. The acidolysis reaction was preformed according to example 2. After 3.5 hours the acidolysis reaction was stopped and the immobilized lipase separated from the reaction mixture by filtration. The immobilized lipase is collected and used for the second run of the acidolysis assay . These runs were repeated eight times. At each run a sample (~ 2 ml) at time 3.5 hours were taken for carbon number analysis.

Table 6 shows the results after acidolysis by reusing the immobilized lipase D on EC-HA support in subsequent 8 runs

**Table 6**

| | Run 1 | Run 2 | Run 3 | Run 4 | Run 5 | Run 6 | Run 7 | Run 8 |
|---|---|---|---|---|---|---|---|---|
| C46 | 0.8 | 1.2 | 1.3 | 1.5 | 1.4 | 1.5 | 1.5 | 1.6 |
| C48 | 11.2 | 19.6 | 22.7 | 25.3 | 24.7 | 26 | 27.7 | 28.5 |
| C50 | 34.1 | 35.9 | 36.4 | 35.9 | 35.8 | 35.6 | 35.3 | 35.2 |
| C52 | 42.4 | 34.2 | 31.3 | 29.4 | 30.1 | 29.1 | 28 | 27.4 |
| C54 | 11.2 | 9.1 | 8.2 | 7.8 | 8 | 7.8 | 7.6 | 7.3 |

### Example 6

Lipase D solution (0.9 g/77 ml) was mixed with various Tween in amounts provided in Table 7 and stirred for 15 min. To each of the preparations 1,5 g of Sepabead EC-HA was added and the mixture was stirred for 24 hours. Then immobilized enzyme was filtered off and tested in the acidolysis reaction as described in example 2. Table 7 shows the results of 5 different Tween's after acidolysis after 3.5 h.

**Table 7**

| Carbonnumber | Tween 20 | Tween 40 | Tween 60 | Tween 80 | Tween 85 |
|---|---|---|---|---|---|
| Amount in g | 0.650 | 0.676 | 0.693 | 0.694 | 0.974 |
| C46 | 1.5 | 2.14 | 2.79 | 1.34 | 2.4 |
| C48 | 16.6 | 41.19 | 54.37 | 18.02 | 46.5 |
| C50 | 34.2 | 31.4 | 27.49 | 33.26 | 30.5 |
| C52 | 36.9 | 18.73 | 11.85 | 36.11 | 15.8 |
| C54 | 10.6 | 5.78 | 3.48 | 10.77 | 4.5 |
| C56 | 0.3 | 0.53 | 0 | 0.38 | 0.3 |

### Example 7

Example 6 was repeated with Tween 80^{®} with the difference that the premixing of the lipase solution with Tween 80^{®} was skipped. Lipase solution, Tween 80^{®} and support material were put together and the mixture was stirred for 24 hours. Then immobilized lipase was filtered off and tested in the acidolysis reaction as described in example 2. Table 8 shows the results after acidolysis after 3.5 h.

**Table 8**

| Carbonnumber | With premixing | No premixing |
|---|---|---|
| C46 | 1.3 | 1.51 |
| C48 | 13.8 | 16.38 |
| C50 | 33 | 32.32 |
| C52 | 40.2 | 38.34 |
| C54 | 11.4 | 11.14 |
| C56 | 0.3 | 0.3 |

The results demonstrate that premixing of Tween 80^{®} with lipase solution is not required.

## Claims

1. A process for producing a triglyceride having a symmetrical structure ABA, which comprises:
immobilizing a 1,3 specific lipase on a support having a functional amino group, wherein the process for immobilizing the lipase on the support comprises contacting the lipase with the support in the presence of a surface-active material, which is a non-ionic surfactant, and is selected from the group consisting of mixtures of polyethyleneglycol and polysorbate; and producing the triglyceride by enzymatic transesterification using the immobilized lipase.

2. A process according to claim 1, wherein the symmetrical triglyceride is Oleic acid / Palmitic acid / Oleic acid (OPO).

3. A process according to claim 1 wherein the symmetrical triglyceride is Stearic or Palmitic acid / Oleic acid / Stearic or Palmitic acid (SOS).

4. A process according to any one of claims 1 to 2, wherein the change of C52 carbon number triglycerides of feedstock to product is at least 15% by weight.

5. A process according to any one of the preceding claims, wherein the lipase is derived from *Rhizopus oryzae.*

6. A process according to any one of the preceding claims, wherein the functional amino group is a functional alkylamino group having C1-C22 carbon atoms.

7. Use of an immobilized lipase, obtainable by a process which comprises contacting a 1,3 specific lipase with a support having a functional amino group, in the presence of a surface-active material selected from the group consisting of mixtures of polyethyleneglycol and polysorbate, for producing a triglyceride fat composition comprising at least 15% by weight OPO.

## Patentansprüche

1. Prozess zur Herstellung eines Triglycerids mit einer symmetrischen Struktur ABA, welcher Folgendes umfasst:
Immobilisieren einer 1,3-spezifischen Lipase auf einem Träger mit einer funktionellen Aminogruppe, wobei das Verfahren zum Immobilisieren der Lipase auf dem Träger das Inkontaktbringen der Lipase mit dem Träger in Gegenwart eines oberflächenaktiven Materials, das ein nicht-ionisches Tensid ist und aus der Gruppe ausgewählt ist, die aus Mischungen von Polyethylenglykol und Polysorbat besteht, und das Herstellen des Triglycerids durch enzymatische Umesterung unter Verwendung der immobilisierten Lipase umfasst.

2. Prozess nach Anspruch 1, wobei das symmetrische Triglycerid Ölsäure/Palmitinsäure/Ölsäure (OPO) ist.

3. Prozess nach Anspruch 1, wobei das symmetrische Triglycerid Stearin- oder Palmitinsäure / Ölsäure / Stearin- oder Palmitinsäure (SOS) ist.

4. Prozess nach einem der Ansprüche 1 bis 2, wobei die Veränderung der C52-Kohlenstoffzahl-Triglyceride vom Ausgangsmaterial zum Produkt mindestens 15 Gew.-% beträgt.

5. Prozess nach einem der vorstehenden Ansprüche, wobei die Lipase von Rhizopus oryzae abgeleitet ist.

6. Prozess nach einem der vorstehenden Ansprüche, wobei die funktionelle Aminogruppe eine funktionelle Alkylaminogruppe mit C1-C22-Kohlenstoffatomen ist.

7. Verwendung einer immobilisierten Lipase, die durch ein Verfahren erhältlich ist, das das Inkontaktbringen einer 1,3-spezifischen Lipase mit einem Träger mit einer funktionellen Aminogruppe in Gegenwart eines oberflächenaktiven Materials umfasst, das aus der Gruppe ausgewählt ist, die aus Mischungen von Polyethylenglykol und Polysorbat besteht, zur Herstellung einer Triglyceridfettzusammensetzung, die mindestens 15 Gew.-% OPO umfasst.

## Revendications

1. Procédé de production d'un triglycéride ayant une structure symétrique ABA, qui comprenant :
immobilisation d'une lipase spécifique 1,3 sur un support ayant un groupe amino fonctionnel, dans lequel le procédé d'immobilisation de la lipase sur le support comprend à mettre en contact de la lipase avec le support en présence d'un matériau tensioactif, qui est un agent de surface non ionique, choisi dans le groupe constitué de mélanges de polyéthylèneglycol et de polysorbate ; et produire le triglycéride par transestérification enzymatique à l'aide de la lipase immobilisée.

2. Procédé selon la revendication 1, dans lequel le triglycéride symétrique est l'acide oléique / l'acide palmitique / l'acide oléique (OPO).

3. Procédé selon la revendication 1, dans lequel le triglycéride symétrique est l'acide stéarique ou palmitique / l'acide oléique / l'acide stéarique ou palmitique (SOS).

4. Procédé selon l'une quelconque des revendications 1 à 2, dans lequel la variation du nombre de carbone C52 entre les triglycérides de la matière première et ceux du produit est d'au moins 15 % en poids.

5. Procédé selon l'une quelconque des revendications précédentes, dans lequel la lipase est dérivée de Rhizopus oryzae.

6. Procédé selon l'une quelconque des revendications précédentes, dans lequel le groupe amino fonctionnel est un groupe alkylamino fonctionnel ayant des atomes de carbone de C1-C22.

7. Utilisation d'une lipase immobilisée, obtenue par un procédé comprenant la mise en contact d'une lipase spécifique 1,3 avec un support ayant un groupe amino fonctionnel, en présence d'un matériau tensioactif choisi dans le groupe constitué de mélanges de polyéthylèneglycol et de polysorbate, pour produire une composition de graisse triglycéridique comprenant au moins 15 % en poids d'OPO.
